# FASCICULE DE BREVET EUROPEEN

(11) **EP 0 679 628 B1**
(45) Date de publication et mention de la délivrance du brevet: **22.04.1998**
(21) Numéro de dépôt: 95400700.1
(22) Date de dépôt: 29.03.1995
(51) Int. Cl.: C07C 59/54, A61K 31/20, C07C 57/52, C07C 59/62, C07C 59/64, C07C 57/42

(54) **Composés polyéniques, compositions pharmaceutiques et cosmétiques les contenant et utilisations**
Polyenderivate, diese enthaltende pharmazeutische und kosmetische Zusammensetzungen und ihre Verwendung
Polyene derivatives, pharmaceutical and cosmetic composition containing them and their use

(30) Priorité: 26.04.1994 FR 9405017
(43) Date de publication de la demande: 02.11.1995
(73) Titulaire: CENTRE INTERNATIONAL DE RECHERCHES DERMATOLOGIQUES GALDERMA (C.I.R.D. GALDERMA), F-06560 Valbonne (FR)
(72) Inventeur: Bernardon, Jean-Michel, F-06650 Le Rouret (FR); Nedoncelle, Philippe, F-06130 Grasse (FR)
(74) Mandataire: Tezier Herman, Béatrice

(56) Documents cités:
- EP-A- 0 486 885
- EP-A- 0 641 759
- FR-A- 2 390 428
- US-A- 3 882 157
- US-A- 4 201 727
- JOURNAL OF ORGANIC CHEMISTRY., vol.43, no.26, 22 Décembre 1978, EASTON US pages 5018 - 5020 BABU A.PATEL ET AL. 'PALLADIUM-CATALYZED ARYLATION OF CONJUGATED DIENES'

## Description

La présente invention concerne, à titre de produits industriels nouveaux et utiles, des composés polyéniques. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies. Les composés selon l'invention peuvent par ailleurs trouver une application intéressante dans le traitement de l'ostéoporose, ou dans le traitement des maladies virales, ainsi que dans le traitement de tout état associé à une hypervitaminose A. D'une manière générale, ils peuvent enfin trouver une application dans le traitement de toute maladie qui est associée à une modification de l'expression des récepteurs appartenant à la superfamille des récepteurs des hormones stéroïdiennes et thyroïdiennes.

On peut également utiliser les composés selon l'invention dans des compositions cosmétiques pour l'hygiène corporelle et capillaire.

Ainsi, une demande EP 0641759, uniquement opposable au titre de l'Article 54(3)CBE, décrit des composés polyéniques présentant le même type d'activité biologique, mais de structures chimiques différentes soit par la longueur de la chaîne polyénique, soit par les substituants du radical phényl présent dans ces composés.

La demande FR-A-2 390 428 décrit des composés polyéniques présentant aussi une activité dermatologique, mais de structures chimiques différentes par la substitution du radical phényl.

Les composés selon l'invention peuvent être représentés par la formule générale (I) suivante : 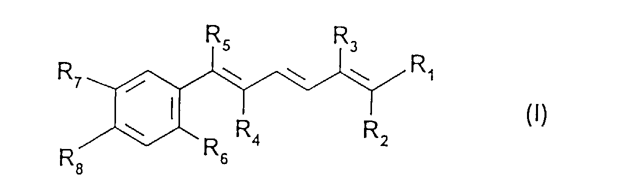 dans laquelle :
* R₁ représente un radical -CH₃, un radical -CH₂-O-R₉, un radical -CH₂-O-CO-R₁₀, ou encore un radical -CO-R₁₁, les différents radicaux R₉, R₁₀ et R₁₁ ayant la signification donnée ci-après,
* R₂ représente un atome d'hydrogène ou un radical alkyle inférieur de C₁-C₆,
* R₃ représente un atome d'hydrogène ou un radical alkyle inférieur de C₁-C₆,
* R₄ représente un atome d'hydrogène,
* R₅ représente un atome d'hydrogène ou un radical alkyle inférieur de C₁-C₆,
* R₆ représente un atome d'hydrogène, un radical alkyle, linéaire ou ramifié, ayant de 1 à 20 atomes de carbone, un radical -O-CH₂-O-CH₂-CH₂-O-CH₃ ou bien encore un radical -O-R₁₃, R₁₃ ayant la signification donnée ci-après,
* R₇ et R₈ représentent indépendamment (i) un atome d'hydrogène, (ii) un radical alkyle, linéaire ou ramifié, ayant de 1 à 20 atomes de carbone, (iii) un radical cycloaliphatique, (iv) un radical -O-CH₂-O-CH₂-CH₂-O-CH₃, (v) un radical -O- R₁₃, ou bien encore (vi) un radical -S(O)ₙR₁₃, R₁₃ et n ayant la signification donnée ci-après,
étant entendu que dans tout ce qui précède :
- R₄ et R₆, pris ensemble, peuvent former avec le cycle benzénique adjacent un cycle naphtalénique,
- l'un au moins des radicaux R₇ ou R₈ représente un radical (ii) ou (iii), avec la condition que lorsque l'un représente le radical (ii), l'autre ne représente pas un radical (ii),
- R₉ représente un atome d'hydrogène ou un radical alkyle inférieur de C₁-C₆
- R₁₀ représente un radical alkyle inférieur de C₁-C₆,
- R₁₁ représente :
   (a) un atome d'hydrogène
   (b) un radical alkyle inférieur de C₁-C₆
   (c) un radical de formule : 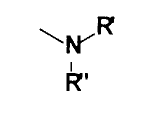 dans laquelle R' et R" représentent indépendamment un atome d'hydrogène, un radical alkyle inférieur de C₁-C₆, un radical mono ou polyhydroxyalkyle, un radical aryle éventuellement substitué ou un reste d'aminoacide ou de peptide ou de sucre ou encore, pris ensemble, forment un hétérocycle,
   (d) un radical -OR₁₂, R₁₂ représentant un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- R₁₃ représente un atome d'hydrogène ou un radical alkyle, linéaire ou ramifié, ayant de 1 à 20 atomes de carbone,
- et n est un nombre entier égal à 0, 1 ou 2.

L'invention vise également les sels des composés de formule (I) ci-dessus dans le cas où R₁ représente une fonction acide carboxylique, ou lorsque R₇ et/ou R₈ représentent une fonction sulfonique, ainsi que les isomères optiques et géométriques desdits composés. Lorsque les composés selon l'invention se présentent sous forme de sels, il s'agit de préférence de sels d'un métal alcalin ou alcalino-terreux, ou encore de zinc ou d'une amine organique.

Selon la présente invention, on entend par radical alkyle inférieur un radical ayant de 1 à 6 atomes de carbone, de préférence les radicaux méthyle, éthyle, isopropyle, butyle, tertiobutyle et hexyle.

Par radical alkyle linéaire ou ramifié ayant de 1 à 20 atomes de carbone, on entend notamment les radicaux méthyle, éthyle, propyle, 2-éthyl-hexyle, octyle, dodécyle, hexadécyle et octadécyl.

Par radical cycloaliphatique, on entend un radical mono ou polycyclique tel que par exemple le radical 1-méthyl cyclohexyle ou le radical 1-adamantyle.

Par radical monohydroxyalkyle, on entend un radical ayant de préférence 2 ou 3 atomes de carbone, notamment un radical 2-hydroxyéthyle, 2-hydroxypropyle ou 3-hydroxypropyle.

Par radical polyhydroxyalkyle, on entend un radical contenant de préférence de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on entend de préférence un radical phényle éventuellement substitué par au moins un atome d'halogène, un hydroxyle ou une fonction nitro.

Par radical aralkyle, on entend de préférence le radical benzyle ou phénéthyle éventuellement substitué par au moins un atome d'halogène, un hydroxyle ou une fonction nitro.

Par radical alkényle, on entend un radical contenant de préférence de 2 à 5 atomes de carbone et présentant une ou plusieurs insaturations éthyléniques, tel que plus particulièrement le radical allyle.

Par reste de sucre, on entend un reste dérivant notamment de glucose, de galactose ou de mannose, ou bien encore de l'acide glucuronique.

Par reste d'aminoacide, on entend notamment un reste dérivant de la lysine, de la glycine ou de l'acide aspartique, et par reste de peptide on entend plus particulièrement un reste de dipeptide ou de tripeptide résultant de la combinaison d'acides aminés.

Par hétérocycle enfin, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Parmi les composés de formule (I) ci-dessus rentrant dans le cadre de la présente invention, on peut notamment citer les suivants :
Acide *trans* 7-[3-(1-adamantyl)-4-hydroxyphenyl]-3,7-diméthyl-2,4,6-heptatrienoïque
Ethyl *trans* 7-[3-(1-adamantyl)-4-méthoxyphenyl]-3,7-diméthyl-2,4,6-heptatrienoate
Acide *trans* 7-[3-(1-adamantyl)-4-méthoxyphenyl]-3,7-diméthyl-2,4,6-heptatrienoïque
Acide (2Z,4E,6E) 7-[3-(1-adamantyl)-4-méthoxyphenyl]-3,7-diméthyl-2,4,6-heptatrienoïque.
Acide *trans* 5-[-7-(1-adamantyl)-6-méthoxyéthoxyméthoxy-2-naphtyl]-3-méthyll-2,4-pentadienoïque.
Acide *trans* 5-[-7-(1-adamantyl)-6-hydroxy-2-naphtyl]-3-méthyl-2,4-pentadienoïque.
Acide *trans* 5-[-7-(1-adamantyl)-6-méthoxy-2-naphtyl]-3-méthyl-2,4-pentadienoïque.
Acide *trans* 5-[-7-(1-adamantyl)-6-propyloxy-2-naphtyl]-3-méthyl-2,4-pentadienoïque.
Acide *trans* 7-[3-(1-méthylcyclohexyl)-4-hydroxyphenyl]-3,7-diméthyl-2,4,6-heptatrienoïque.
Acide (2Z,4E,6E) 7-[3-(1-méthylcyclohexyl)-4-méthoxyphenyl]-3,7-diméthyl-2,4,6-heptatrienoïque.
Acide *trans* 7-[3-(1-adamantyl)-4-isopropyloxyphenyl]-3,7-diméthyl-2,4,6-heptatrienoïque.
acide *trans* 7-[3-(1-adamantyl)-4-méthoxyéthoxyméthoxyphenyl]-3,7-diméthyl-2,4,6-heptatrienoïque.
acide *trans* 7-[3-(1-adamantyl)-4-méthoxyphenyl]-7-méthyl-2,4,6-heptatrienoïque.
*trans* 7-[3-(1-adamantyl)-4-méthoxyphenyl]-3,7-diméthyl-2,4,6-heptatrien-1-ol.
*trans* 7-[3-(1-adamantyl)4-méthoxyphenyl]-3,7-diméthyl-2,4,6-heptatrien-1-al.
*trans* N-éthyl-7-[3-(1-adamantyl)4-méthoxyphenyl]-3,7-diméthyl-2,4,6-heptatrienecarboxamide.
*trans* N-4-hydroxyphényl-7-[3-(1-adamantyl)-4-méthoxyphenyl]-3,7-diméthyl-2,4,6-heptatrienecarboxamide.
acide *trans* 7-(3-tert-butyl-4-méthoxyéthoxyméthoxyphenyl)-3,7-diméthyl-2,4,6-heptatrienoïque.
*trans* 7-(3-tert-butyl-4-hydroxyphenyl)-3,7-diméthyl-2,4,6-heptatrienoate d'éthyle.
*trans* 7-(3-tert-butyl-4-méthoxyphenyl)-3,7-diméthyl-2,4,6-heptatrienoate d'éthyle.
*trans* 7-[4-(1-adamantyl)-3-méthoxyphenyl]-3,7-diméthyl-2,4,6-heptatrienoate d'éthyle.

Selon la présente invention les composés de formule (I) plus particulièrement préférés sont ceux pour lesquels l'une au moins, et de préférence toutes, les conditions ci-dessous sont respectées :
- R₁ est un radical -CO-R₁₁
- R₇ est le radical adamantyl
- R₄ est un atome d'hydrogène
- R₆ est un atome d'hydrogène ou un radical alkyl, linéaire ou ramifié, ayant de 1 à 20 atomes de carbone
- R₄ et R₆ forment avec le cycle benzénique adjacent un cycle naphthalénique.

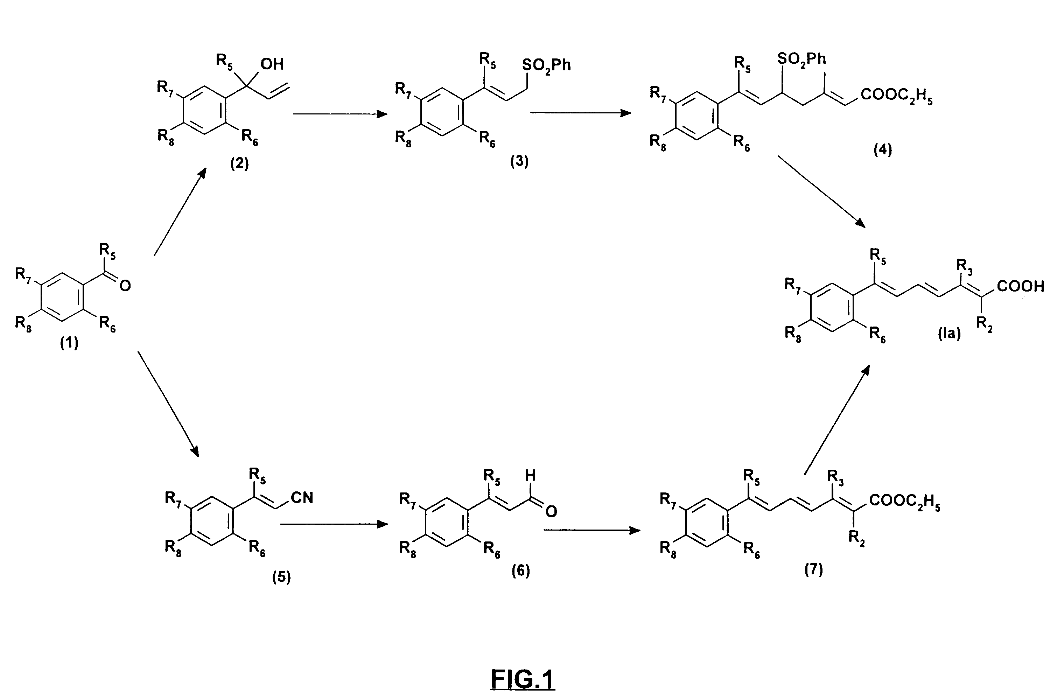

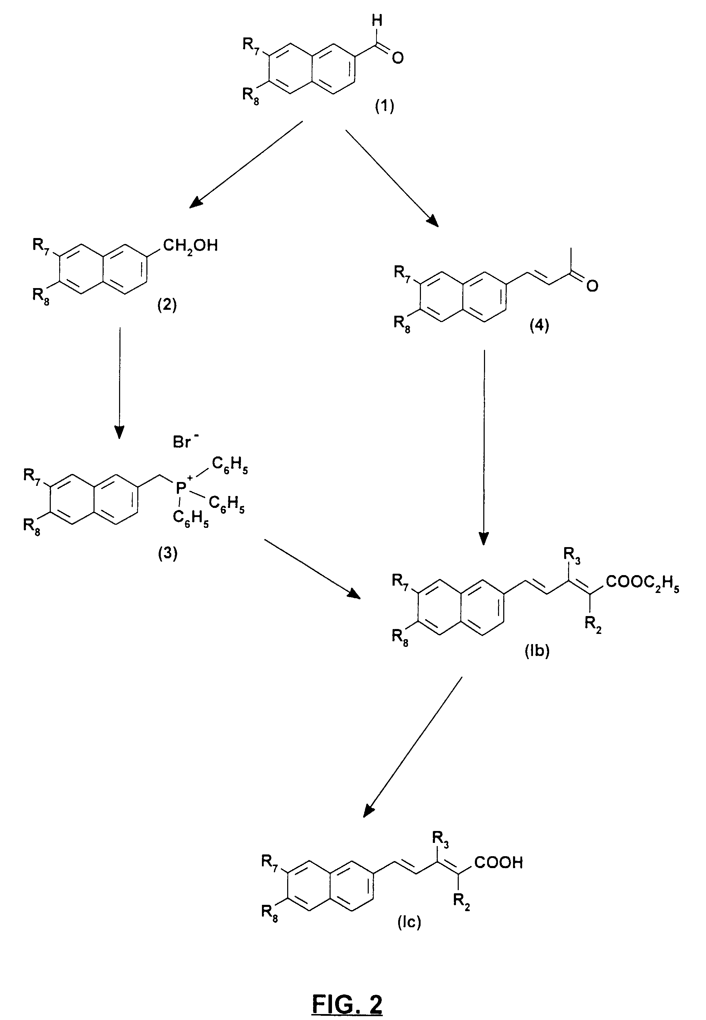

La présente invention a également pour objet les procédés de préparation des composés de formule (I), en particulier selon les schémas réactionnels donnés aux figures 1 et 2.

Les composés de formule I(a) peuvent être ainsi, par exemple, être obtenus selon deux voies (cf. figure 1) :
- soit selon la synthèse décrite par M. Julia et D. Arnould dans Bull. Soc. Chim. (1973) 746. Ainsi, par réaction entre un dérivé acétophenone (1) et du bromure de vinylmagnésium, on prépare l'α-hydroxyvinyl (2) qui est ensuite mis en réaction avec du phénylsulfinate de sodium afin d'obtenir le composé (3). L'anion du dérivé (3) est formé en présence d'une base, par exemple le tert-butylate de potassium, et réagit avec un γ-bromosenecionate d'éthyle afin d'obtenir la sulfone-ester (4). L'élimination du groupement sulfone est éffectuée en présence d'une base, par exemple tert-butylate de potassium ou DBU, et après saponification de la fonction ester dans la soude ou la potasse alcoolique, on obtient enfin l'acide I(a).
- soit par réaction de l'acétophenone (1), selon une réaction de type Horner, avec un C2-phosphononitrile, afin de prérarer le dérivé propenenitrile (5). Après réduction partielle avec de l'hydrure de diisobutylaluminium (DIBAH), on obtient le dérivé propénal (6). Le traitement de cet aldéhyde avec le lithio dérivé d'un phosphonate d'ester aboutit à la formation de l'ester (7) qui, par saponification dans la soude ou la potasse alcoolique, donne enfin l'acide I(a).

Les composés de formule I(b) et I(c) peuvent, quant à eux, être obtenus comme suit (cf. figure 2) :
- soit à partir de l'aldéhyde (1) par réduction en présence d'un hydrure alcalin, tel que le borohydrure de sodium ou l'hydrure double de lithium et d'aluminium. L'alcool (2) ainsi obtenu est alors transformé en sel de phosphonium (3) par réaction avec (C₆H₅)₃P.HBr dans le méthanol, ce composé (3) étant ensuite converti en ester de formule I(b) par action du lithio ou sodio dérivé d'un phosphonate d'ester.
- soit à partir de l'aldéhyde (1) selon une suite de deux réactions de type Horner. Tout d'abord, on fait réagir le composé (1) avec le diméthyl-2-oxopropylphosphonate en présence de DBU dans le THF pour obtenir la cétone (4), puis on transforme cette dernière en dérivé ester I(b) par réaction avec le lithio ou sodio dérivé d'un phosphonate d'ester. Par saponification de l'ester I(b) en présence de soude ou potasse alcoolique, on obtient finalement l'acide I(c).

Lorsque R₆, R₇ et R₈ représentent le radical hydroxy, les composés de formule (I) sont avantageusement préparés en protégeant la fonction phénol, de préférence sous forme de tert-butyldimethylsilyloxy ou de méthoxéthoxyméthoxy, la déprotection étant alors effectuée soit en présence de fluorure de tetrabutylammonium ou d'iodure de triméthylsilyl soit en milieu acide (HCI).

La présente invention a également pour objet à titre de médicament les composés de formule (I) telle que définie ci-dessus.

Ces composés présentent une activité antagoniste vis à vis de l'expression d'un ou plusieurs marqueurs biologiques dans le test de différenciation des cellules (F9) de tératocarcinome embryonnaire de la souris (Skin Pharmacol., 3, pp 256-267, 1990) et/ou sur la différenciation des kératinocytes humains in vitro (Anal. Biochem., 192, pp 232-236, 1991). Ils peuvent en outre présenter une activité agoniste spécifique vis à vis de certains récepteurs, et antagonistes pour d'autres.

Les composés selon l'invention conviennent particulièrement bien dans les domaines de traitement suivants :
1) pour traiter les affections dermatologiques liées à un désordre de la kératinisation portant sur la différenciation et sur la prolifération notamment pour traiter les acnées vulgaires, comédoniennes, polymorphes, rosacées, les acnées nodulokystiques, conglobata, les acnées séniles, les acnées secondaires telles que l'acnée solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acnée ou la séborrhée simple,
11) dans le traitement ou la prévention des états cancéreux ou précancéreux,
12) dans le traitement d'affections inflammatoires telles que l'arthrite,
13) dans le traitement de toute affection d'origine virale au niveau cutané ou général,
14) dans la prévention ou le traitement de l'alopécie,
15) dans le traitement d'affections dermatologiques ou générales à composante immunologique,
16) dans le traitement d'affections du système cardiovasculaire telles que l'artériosclérose,
17) dans le traitement ou la prévention de l'ostéoporose.

Dans les domaines thérapeutiques mentionnés ci-dessus, les composés selon l'invention peuvent être avantageusement employés en combinaison avec d'autres composés actifs, par exemple avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase, l'Ubiquinol ou certains chélatants de métaux. Par α-hydroxy ou α-céto acides ou leurs dérivés, on entend par exemple les acides lactique, malique, citrique, glycolique, mandélique, tartrique, glycérique ou ascorbique ou leurs sels, amides ou esters. Enfin, par bloqueurs de canaux ioniques, on entend par exemple le Minoxidil (2,4-diamino-6-pipéridino-pyrimidine 3-oxyde) et ses dérivés.

La présente invention a également pour objet des compositions médicamenteuses contenant au moins un composé de formule (I) telle que définie ci-dessus, l'un de ses isomères optiques ou géométriques ou un de ses sels.

La présente invention a donc ainsi pour objet une nouvelle composition médicamenteuse destinée notamment au traitement des affections susmentionnées, et qui est caractérisée par le fait qu'elle comprend, dans un support pharmaceutiquement acceptable et compatible avec le mode d'administration retenu pour cette dernière, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/Kg en poids corporel, et ceci à raison de 1 à 3 prises.

Par voie topique, les compositions pharmaceutiques à base de composés selon l'invention sont plus particulièrement destinées au traitement de la peau et des muqueuses et peuvent alors se présenter sous forme d'onguents, de crêmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent par ailleurs se présenter soit sous forme anhydre, soit sous une forme aqueuse, selon l'indication clinique. Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001% et 5% en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photo-induit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques, tous ces différents produits étant tels que définis ci-avant.

La présente invention vise donc également une composition cosmétique qui est caractérisée par le fait qu'elle comprend, dans un support cosmétiquement acceptable et convenant à une application topique, au moins un composé de formule (I) telle que définie ci-dessus ou l'un de ses isomères optiques ou géométriques ou l'un de ses sels, cette composition cosmétique pouvant notamment se présenter sous la forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques selon l'invention est avantageusement comprise entre 0,001% et 3% en poids par rapport à l'ensemble de la composition.

Les compositions médicamenteuses et cosmétiques selon l'invention peuvent en outre contenir des additifs inertes ou même pharmacodynamiquement ou cosmétiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolidones-3; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,5-diphényl-imidazolidine 2,4-dione); des agents anti-inflammatoires non stéroïdiens; des caroténoïdes et, notamment, le β-carotène ; des agents anti-psoriatiques tels que l'anthraline et ses dérivés; et enfin les acides eicosa-5,8,11,14-tétraynoïque et eicosa-5,8,11-triynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'a-tocophérol, le butylhydroxyanisole ou le butylhydroxytoluène.

On va maintenant donner, à titre d'illustration et sans aucun caractère limitatif, plusieurs exemples d'obtention de composés actifs de formule (I) selon l'invention, ainsi que diverses formulations concrètes à base de tels composés.

### EXEMPLE 1

### Acide trans 7-[3-(1-adamantyl)-4-hydroxyphenyl]-3,7-diméthyl-2,4,6-heptatrienoïque

(a) 2-[3-(1-adamantyl)-4-tert-butyldiméthylsilyloxyphenyl]-3-butène-2-ol
   Dans un tricol, on introduit 385 mg (1 mmole) de 3-(1-adamantyl)-4-tert-butyldimethylsilyloxyacetophenone et 20 ml de THF, puis on ajoute goutte à goutte 1,1 ml (1,1 mmole) d'une solution de bromure de vinylmagnésium (1M) de manière à ce que la température du milieu réactionnel ne dépasse pas 20°C. Puis on agite à température ambiante pendant une heure, verse le milieu réactionnel dans un mélange glace-eau, extrait avec de l'éther éthylique, décante la phase organique, sèche sur sulfate de magnésium, évapore. Le résidu obtenu est purifié par chromatographie sur colonne de silice élué avec un mélange d'éther éthylique et d'hexane (15-85). On recueille ainsi 380 mg (rendement : 92%) de l'alcool attendu sous forme d'une huile.
(b) 3-[3-(1-adamantyl)-4-tert-butyidiméthylsilyloxyphenyl]-2-butènesulfonylbenzene
   Dans un ballon, on introduit 5,9 g (35,8 mmoles) de benzenesulfinate de sodium, 8 ml d'eau et 12 ml d'acide acétique. On chauffe à 60°C, on ajoute ensuite en quinze minutes 7,37 g (17,8 mmoles) du dérivé alcoolique obtenu précédemment et on maintient cette température pendant quatre heures. On verse le milieu réactionnel ainsi obtenu dans de l'eau, extrait avec de l'hexane, décante la phase organique, lave à l'eau, sèche sur sulfate de magnésium, et évapore. Le résidu obtenu est purifié par chromatographie sur colonne de silice élué avec un mélange d'acétate d'éthyle et d'hexane (10-90). Après évaporation des solvants, on recueille 3,1 g (rendement: 32%) du produit attendu.
(c) Ethyl *trans* 7-[3-(1-adamantyl)-4-tert-butyidiméthylsilyloxyphenyl]-5-benzenesulfonyl-3,7-diméthyl-2,6-heptadienoate
   Dans un ballon, on introduit 765 mg (6,8 mmoles) de tert-butylate de potassium et 5 ml d'une solution constituée d'un mélange de THF et de N-methylpyrrolidine (5-1). Tout en refroidissant l'ensemble à -65°C, on ajoute alors au mélange ainsi obtenu, sucessivement et goutte à goutte, d'abord une première solution constituée de 3 g (5,68 mmoles) du dérivé obtenu au point (b) dilué dans 2,5 ml d'un mélange [THF/N-méthylpyrrolidine] identique à celui indiqué ci-avant, puis une deuxième solution constituée de 1,3 g (6,25 mmoles) de γ-bromosenecionate d'éthyle dilué dans 1 ml de THF, et agite le tout une heure à 65°C.
   On ajoute ensuite 25 ml d'éther éthylique, laisse remonter la température à 0°C, ajoute 35 ml d'eau et extrait avec de l'éther éthylique. On décante la phase organique, sèche sur sulfate de magnésium, puis évapore. Le résidu obtenu est purifié par chromatographie sur colonne de silice élué avec un mélange d'acétate d'éthyle et d'hexane (15-85). Après évaporation, on recueille 4 g (rendement : 100%) de l'ester attendu sous forme d'une huile incolore.
(d) Ethyl *trans* 7-[3-(1-adamantyl)-4-hydroxyphenyl]-3,7-diméthyl-2,4,6-heptatrienoate
   Dans un ballon et sous courant d'azote, on introduit 4 g (6,25 mmoles) de l'ester précédent obtenu au point (c) et 40 ml de THF, on refroidit l'ensemble à 30°C et on ajoute 700 mg (6,25 mmoles) de tert-butylate de potassuim. On agite à cette même température pendant une heure, laisse remonter à température ambiante, neutralise le milieu réactionnel et extrait avec de l'éther éthylique. On décante la phase organique, sèche sur sulfate de magnèsium, évapore. Le résidu obtenu est purifié par chromatographie sur colonne de silice élué avec un mélange d'acétate d'éthyle et d'hexane (20-80). On recueille, après évaporation des solvants, 863 mg (rendement : 34%) d'ethyl 7-[3-(1-adamantyl)-4-hydroxyphenyl]-3,7-diméthyl-2,4,6-heptatrienoate sous forme d'une huile.
(e) Acide *trans* 7-[3-(1-adamantyl)-4-hydroxyphenyl]-3,7-diméthyl-2,4,6-heptatrienoïque
   Dans un ballon à l'abri de la lumière et sous courant d'azote, on introduit 813 mg (2 mmoles) de l'ester obtenu au point (d) précédent et 50 ml de méthanol. On ajoute goutte à goutte 2,5 ml (10 mmoles) de soude (4N) et on chauffe à reflux pendant deux heures. Après refroidissement, on ajoute de l'acétate d'éthyle, acidifie avec de l'acide chlorhydrique (4N), décante la phase organique, lave à l'eau, sèche sur sulfate de magnèsium, et évapore. Le résidu obtenu est purifié par chromatographie sur colonne de silice élué avec un mélange de THF et d'hexane (30-70). Après évaporation des solvants, on recueille 400 mg (rendement : 53%) d'acide 7-[3-(1-adamantyl)-4-hydroxyphenyl]-3,7-diméthyl-2,4,6-heptatrienoïque de point de fusion 210°C avec décomposition.

### EXEMPLE 2

### Ethyl trans 7-[3-(1-adamantyl)-4-méthoxyphenyl]-3,7-diméthyl-2,4,6-heptatrienoate

Dans un ballon et sous courant d'azote, on introduit 813 mg (2 mmoles) de l'ester obtenu à l'exemple 1(d) et 30 ml de THF. On ajoute par petites quantités 60 mg (2 mmoles) d'hydrure de sodium (80% dans l'huile) et agite jusqu'à cessation du dégagement gazeux. On ajoute ensuite 125 µl (2 mmoles) d'iodure de méthyle et agite à température ambiante trois heures. On verse le milieu réactionnel dans l'eau, extrait avec de l'éther éthylique, décante la phase organique, sèche sur sulfate de magnésium, et évapore. Le résidu obtenu est purifié sur colonne de silice élué avec un mélange de dichlorométhane et d'hexane (50-50). On recueille 714 mg (rendement : 85%) de l'ester attendu sous forme d'une huile.

### EXEMPLE 3

### Acide trans 7-[3-(1-adamantyl)-4-méthoxyphenyl]-3,7-diméthyl-2,4,6-heptatrienoïque

(a) 2-[3-(1-adamantyl)-4-méthoxyphenyl]-3-butène-2-ol
   De manière analogue à l'exemple 1(a), à partir de 12,7 g (44,6 mmoles) de 3-(1-adamantyl)-4-méthoxyacetophenone et après chromatographie sur colonne de silice élué avec un mélange d'acétate d'éthyle et d'hexane (10-90), on obtient 7,6 g (rendement : 54%) de l'alcool attendu sous forme d'une huile jaune.
(b) 3-[3-(1-adamantyl)-4-méthoxyphenyl]-2-butènesulfonylbenzene
   De manière analogue à l'exemple 1(b), à partir de 7,6 g (24,2 mmoles) de l'alcool obtenu au point (a) ci-dessus et après chromatographie sur colonne de silice élué avec un mélange d'acétate d'éthyle et d'hexane (15-85), on obtient 5,2 g (rendement: 49%) du produit attendu.
(c) Ethyl *trans* 7-[3-(1-adamantyl)4-méthoxyphenyl]-5-benzenesulfonyl-3,7-diméthyl-2,6-heptadienoate
   De manière analogue à l'exemple 1(c), à partir de 5,18 g (11,9 mmoles) du 3-[3-(1-adamantyl)-4-méthoxyphenyl]-2-butènesulfonylbenzene obtenu au point (b) ci-dessus et après chromatographie sur colonne de silice élué avec un mélange d'acétate d'éthyle et d'hexane (20-80), on obtient 5 g (75%) de l'ester attendu.
(d) Acide *trans* 7-[3-(1-adamantyl)-4-méthoxyphenyl]-3,7-diméthyl-2,4,6-heptatrienoïque
   Dans un ballon à l'abri de la lumière et sous courant d'azote, on introduit 4,6 g (8,2 mmoles) de l'ester préparé au point (c) précédent et 50 ml de méthanol. Puis, on ajoute goutte à goutte 23 ml (98 mmoles) de potasse méthanolique (4N) et chauffe à reflux pendant deux heures. Après refroidissement, on ajoute de l'éther éthylique et de l'eau, acidifie avec de l'acide sulfurique (4N), décante la phase organique, lave à l'eau, sèche sur sulfate de magnèsium, et évapore. Le résidu obtenu est purifié par chromatographie sur colonne de silice élué avec un mélange d'acétate d'éthyle et de dichlorométhane (15-85). Après évaporation des solvants, on recueille 2,3 g (rendement: 72%) d'acide *trans* 7-[3-(1-adamantyl)-4-méthoxyphenyl]-3,7-diméthyl-2,4,6-heptatrienoïque de point de fusion 212°C avec décomposition.

### EXEMPLE 4

### Acide (2Z,4E,6E) 7-[3-(1-adamantyl)-4-méthoxyphenyl]-3,7-diméthyl-2,4,6-heptatrienoïque

Dans la réaction de l'exemple 3 (d), lors de la chromatographie sur colonne de silice élué avec un mélange d'acétate d'éthyle et de dichlorométhane (15-85), on obtient également 310 mg (rendement : 9%) d'acide (2Z,4E,6E) 7-[3-(1-adamantyl)-4-méthoxyphenyl]-3,7-diméthyl-2,4,6-heptatrienoïque de point de fusion 207°C avec décomposition.

### EXEMPLE 5

### Acide trans 5-[-7-(1-adamantyl)-6-méthoxyéthoxyméthoxy-2-naphtyl]-3-méthyl]-2,4-pentadienoïque.

(a) 7-(1-adamantyl)-6-hydroxy-2-bromonaphtalène.
   Dans un ballon, on introduit 22,3 g (0,1 mole) de 6-bromo-2-naphtol 15,2 g (0,1 mole) de 1-adamantanol et 100 ml de dichlorométhane. On ajoute 5,5 ml d'acide chlorhydrique concentré et agite à température ambiante pendant douze heures. On évapore à sec, reprend par l'eau et neutralise avec du bicarbonate de sodium. On extrait avec de l'acétate d'éthyle, décante la phase organique, sèche sur sulfate de magnésium, évapore.Le résidu obtenu est trituré dans un mélange de dichlorométhane et d'heptane (30-70), filtré et séché. On recueille 19 g (53%) du produit attendu de point de fusion 215-6°C.
(b) 7-(1-adamantyl)-6-méthoxyéthoxyméthoxy-2-bromonaphtalène.
   Dans un tricol et sous courant d'azote on introduit 17,85 g (50 mmoles) de 7-(1-adamantyl)-6-hydroxy-2-bromonaphtalène et 200 ml de DMF. On introduit par petites quantités 1,8g (60 mmoles) d'hydrure de sodium (80% dans l'huile) et agite à température ambiante jusqu'à cessation du dégagement gazeux. On refroidit à 5°C et introduit goutte à goutte 6,9 ml (60 mmoles) de chlorure de méthoxyéthoxyméthyle et agite pendant deux heures. On verse le milieu réactionnel dans l'eau glacée, extrait avec de l'éther éthylique, décante la phase organique, sèche sur sulfate de magnésium, évapore. Le résidu obtenu est purifié par chromatographie sur colonne de silice élué avec un mélange de dichlorométhane et d'heptane (40-60). On recueille après évaporation des solvants 19,5 g (87%) du produit attendu de point de fusion 99-100°C.
(c) 7-(1-adamantyl)-6-méthoxyéthoxyméthoxy-2-naphtalènecarboxaldéhyde.
   Dans un tricol et sous courant d'azote, on introduit 15 g (33,7 mmoles) de 7-(1-adamantyl)-6-méthoxyéthoxyméthoxy-2-bromonaphtalène et 150 ml de THF. A -78°C on ajoute goutte à goutte une solution de 14,9 ml de n-butyllithium (2,5 M dans hexane) et agite pendant une heure. A cette même température, on ajoute 3,1 ml (40,4 mmoles) de DMF et laisse remonter à température ambiante. On verse le milieu réactionnel dans une solution aqueuse saturée en chlorure d'ammonium, extrait avec de l'éther éthylique, décante la phase organique, sèche sur sulfate de magnésium, évapore. Le résidu obtenu est purifié par chromatographie sur colonne de silice élué avec un mélange d'acétate d'éthyle et d'heptane (1-4). On recueille après évaporation des solvants 7,7 g (57%) du dérivé aldéhydique attendu de point de fusion 115-6°C.
(d) trans 5-[-7-(1-adamantyl)-6-méthoxyéthoxyméthoxy-2-naphtyl]-3-méthyll-2,4-pentadienoate d'éthyle.
   Dans un tricol et sous courant d'azote, on introduit 3,1 ml (22,2 mmoles) de diisopropylamine 40 ml de HMPA et 60 ml de THF. A 0°C, on introduit goutte à goutte 8,9 ml de n-butyllithium (2,5 M dans l'hexane) et agite pendant 30 min.. A -60°C, on ajoute goutte à goutte une solution de 6,3 ml (20,7 mmoles) de triéthyl 3-méthyl-4-phosphonocrotonate dans 50 ml de THF, agite pendant une heure puis on ajoute goutte à goutte une solution de 6 g (14,8 mmoles) de 7-(1-adamantyl)-6-méthoxyéthoxyméthoxy-2-naphtalènecarboxaldéhyde dissous dans 30 ml de THF et laisse remonter à température ambiante. On verse le mileu réactionnel dans une solution aqueuse saturée en chlorure d'ammonium, extrait avec de l'éther éthylique, décante la phase organique, sèche sur sulfate de magnésium, évapore. Le résidu obtenu est purifié par chromatographie sur colonne de silice élué avec un mélange d'acétate d'éthyle et d'heptane (1-9). Après évaporation des solvants, on recueille 6 g (85%) de l'ester éthylique attendu sous forme d'une huile jaune.
(e) acide trans 5-[-7-(1-adamantyl)-6-méthoxyéthoxyméthoxy-2-naphtyl]-3-méthyll-2,4-pentadienoïque.
   De manière analogue à l'exemple 1(e) à partir de 1,2 g (2,5 mmoles) de l'ester éthylique précédent, on obtient 350 mg (35%) d'acide trans 5-[-7-(1-adamantyl)-6-méthoxyéthoxyméthoxy-2-naphtyl]-3-méthyll-2,4-pentadienoïque de point de fusion 195-6°C

### EXEMPLE 6

### Acide trans 5-[-7-(1-adamantyl)-6-hydroxy-2-naphtyl]-3-méthyll-2,4-pentadienoïque.

(a) trans 5-[-7-(1-adamantyl)-6-hydroxy-2-naphtyl]-3-méthyll-2,4-pentadienoate d'éthyle.
   Dans un tricol et sous courant d'azote, on introduit 5,6 g (11,8 mmoles) de trans 5-[-7-(1-adamantyl)-6-méthoxyéthoxyméthoxy-2-naphtyl]-3-méthyll-2,4-pentadienoate d'éthyle et 60 ml de dichlorométhane. A -70°C on ajoute goutte à goutte 13 ml de trichlorure de bore (1M dans le dichlorométhane) et laisse remonter à température ambiante. On verse le milieu réactionnel dans l'eau glacée, extrait avec de l'éther éthylique, décante la phase organique, sèche sur sulfate de magnésium, évapore. Le résidu obtenu est purifié par chromatographie sur colonne de silice élué avec un mélange d'acétate d'éthyle et d'heptane (1-4). Après évaporation des solvants, on recueille 1,4 g (85%) de l'ester éthylique attendu de point de fusion 212-4°C.
(b) acide trans 5-[-7-(1-adamantyl)-6-hydroxy-2-naphtyl]-3-méthyll-2,4-pentadienoïque.
   De manière analogue à l'exemple 1(e) à partir de 1,3 g (3,1 mmole) de l'ester éthylique précédent, on obtient 320 mg (25%) acide trans 5-[-7-(1-adamantyl)-6-hydroxy-2-naphtyl]-3-méthyll-2,4-pentadienoïque de point de fusion 240-5°C.

### EXEMPLE 7

### Acide trans 5-[-7-(1-adamantyl)-6-méthoxy-2-naphtyl]-3-méthyll-2,4pentadienoïque.

(a) trans 5-[-7-(1-adamantyl)-6-méthoxy-2-naphtyl]-3-méthyll-2,4-pentadienoate d'éthyle.
   De manière analogue à l'exemple 2 par réaction de 1,4 g (3,3 mmoles) de trans 5-[-7-(1 -adamantyl)-6-hydroxy-2-naphtyl]-3-méthyll-2,4-pentadienoate d'éthyle avec 260 µl (4 mmoles) d'iodure de méthyle, on obtient 330 mg (80%) d'ester éthylique attendu.
(b) acide trans 5-[-7-(1-adamantyl)-6-méthoxy-2-naphtyl]-3-méthyll-2,4-pentadienoïque.
   De manière analogue à l'exemple 1(e) à partir de 1,1 g (2,7 mmoles) de l'ester éthylique précédent, on obtient 478 mg (43%) d' acide trans 5-[-7-(1-adamantyl)-6-méthoxy-2-naphtyl]-3-méthyll-2,4-pentadienoïque de point de fusion 290-1°C.

### EXEMPLE 8

### Acide trans 5-[-7-(1-adamantyl)-6-propyloxy-2-naphtyl]-3-méthyll-2,4-pentadienoïque.

(a) trans 5-[-7-(1-adamantyl)-6-propyloxy-2-naphlyl]-3-méthyll-2,4-pentadianoate d'éthyle.
   De manière analogue à l'exemple 2 par réaction de 1,4 g (3,3 mmoles) de trans 5-[-7-(1-adamantyl)-6-hydroxy-2-naphtyl]-3-méthyll-2,4-pentadienoate d'éthyle avec 400 µl (4 mmoles) de 3-iodopropane, on obtient 1,12 g (75%) d'ester éthylique attendu de point de fusion 133-4°C.
(b) acide trans 5-[-7-(1-adamantyl)-6-propyloxy-2-naohtyl)-3-méthyll-2,4-pentadienoïque.
   De manière analogue à l'exemple 1(e) à partir de 1,1 g (2,4 mmoles) de l'ester éthylique précédent, on obtient 770 mg (80%) d' acide trans 5-[-7-(1-adamantyl)-6-propyloxy-2-naphtyl)-3-méthyll-2,4-pentadienoïque de point de fusion 240-1°C.

### EXEMPLE 9

### Acide trans 7-[3- 1-méthylcyclohexyl)-4-hydroxyphenyl]-3,7-diméthyl-2,4,6-heptatrienoïque.

(a) 3-(1-méthylcyclohexyl)-4-méthoxyphényléthanone.
   Dans un tricol et sous courant d'azote, on introduit 4,6 g (1,85 mmoles) d'acide 3-(1-méthylcyclohexyl)-4-méthoxybenzoïque et 50 ml de THF. A 30°C on ajoute goutte à goutte 29 ml (4,6 mmoles) de méthyllithium (1,6M dans l'éther éthylique) et laisse remonter à température ambiante. On verse le milieu réactionnel dans l'eau glacéé, extrait avec de l'acétate d'éthyle, décante la phase organique, sèche sur sulfate de magnésium, évapore. On recueille 4,6 g (99%) du dérivé cétonique attendu sous forme d'une huile incolore.
(b) 3-méthyl-3-[3-(1-méthylcyclohexyl)-4-méthoxyphényl]acrylonitrile.
   On dissout 4,5 g (18,3 mmoles) de 3-(1-méthylcyclohexyl)-4-méthoxyphényléthanone dans 50 ml de THF et ajoute successivement 3,2 g (27 mmoles) de cyanométhylphosphonate de diéthyle 2,4 g de potasse broyé. On agite à température ambiante pendant quatre heures. On verse le milieu réactionnel dans l'eau glacée, extrait avec de l'acétate d'éthyle, décante la phase organique, sèche sur sulfate de magnésium, évapore. Le résidu obtenu est purifié par chromatographie sur colonne de silice élué avec un mélange de dichlorométhane et d'heptane (50-50). On recueille 2,86 g (58%) du nitrile attendu.
(c) 3-[3-(1-méthylcyclohexyl)-4-méthoxyphényl]crotonaldéhyde.
   On dissout 2,8 g (10,4 mmoles) du composé précédent dans 50 ml de toluène anhydre et à -70°C, on ajoute goutte à goutte 13 ml (13 mmoles) d'une solution d'hydrure de diisobutyl aluminium (1M dans le toluène). On laisse revenir à 0°C et agite pendant 30 minutes. On ajoute lentement de l'acide chlorhydrique dilué puis filtre sur celite. Le filtrat est lavé à l'eau, sèché sur sulfate de magnésium, évaporé. Le résidu obtenu est purifié par chromatographie sur colonne de silice élué avec un mélange de dichlorométhane et d'heptane (70-30). On recueille 2,34 g (83%) de l'aldéhyde attendu.
(d) trans 7-[3-(1-méthylcyclohexyl)-4-hydroxyphenyl]-3,7-diméthyl-2,4,6-heptatrienoate d'éthyle.
   De manière analogue à l'exemple 5(d) par réaction de 2,3 g (8,6 mmoles) de 3-[3-(1-méthylcyclohexyl)-4-méthoxyphényl]crotonaldéhyde avec 4,4 ml (12,8 mmoles) de 3-méthyl-4-phosphono-2-butenoate de triéthyle, on obtient 2,13 g (65%) de l'ester éthylique attendu sous forme d'une huile jaune.
(e) acide trans 7-[3-(1-méthylcyclohexyl)-4-hydroxyphenyl]-3,7-diméthyl-2,4,6-heptatrienoïque.
   De manière analogue à l'exemple 1(e) à partir de 2,13 g (5,5 mmoles) de l'ester éthylique précédent, on obtient après chromatographie sur colonne de silice élué avec du dichlorométhane 540 mg (27%) d' acide trans 7-[3-(1-méthylcyclohexyl)-4-hydroxyphenyl]-3,7-diméthyl-2,4,6-heptatrienoïque de point de fusion 175-7°C.

### EXEMPLE 10

### Acide (2Z,4E,6E) 7-[3-(1-méthylcyclohexyl)-4-méthoxyphenyl]-3,7-diméthyl-2,4,6-heptatrienoïque.

Dans la réaction de l'exemple 9(e) lors de la chromatographie sur colonne de silice élué avec du dichlorométhane, on obtient aussi 100 mg (5%) de l'acide (2Z,4E,6E) 7-[3-(1-méthylcyclohexyl)-4-méthoxyphenyl]-3,7-diméthyl-2,4,6-heptatrienoïque de point de fusion 165-167°C.

### EXEMPLE 11

### Acide trans 7-[3-(1-adamantyl)-4-isopropyloxyphenyl]-3,7-diméthyl-2,4,6-heptatrienoïque.

(a) 3-(1-adamantyl)-4-isopropyloxyphényléthanone.
   De manière analogue à l'exemple 9(a) par réaction de 5 g (16 mmoles) d'acide 3-(1-adamantyl)-4-isopropyloxybenzoïque avec 25 ml (44 mmoles) de méthyllithuim (1,6M), on obtient 4,9 g (98%) du dérivé cétonique attendu de point de fusion 101-2°C.
(b) 3-méthyl-3-[3-(1-adamantyl)-4-isopropyloxyphényl]acrylonitrile.
   De manière analogue à l'exemple 9(b) par réaction de 3,6 g (11,5 mmoles) de la cétone précédente avec 2,03 g (17,3 mmoles) de cyanométhylphosphonate de diéthyle, on obtient 2,07 g (54%) de nitrile attendu.
(c) 3-[3-(1-adamantyl)-4-isopropyloxyphényl]crotonaldéhyde.
   De manière analogue à l'exemple 9(c) à partir de 3,1 g (9,2 mmoles) du nitrile précédent, on obtient 2,28 g (73%) de l'aldéhyde attendu.
(d) trans 7-[3-(1-adamantyl)-4-isopropyloxyphenyl]-3,7-diméthyl-2,4,6-heptatrienoate d'éthyle.
   De manière analogue à l'exemple 5(d) par réaction de 1,37 g (4 mmoles) de 3-[3-(1-adamantyl)-4-isopropyloxyphényl]crotonaldéhyde avec 1,92 ml (6 mmoles) de 3-méthyl-4-phosphono-2-butenoate de triéthyle, on obtient 1 g (57%) de l'ester éthylique attendu sous forme d'une huile jaune.
(e) acide trans 7-[3-(1-adamantyl)-4-isopropyloxyphenyl]-3,7-diméthyl-2,4,6-heptatrienoïque.
   De manière analogue à l'exemple 1(e) à partir de 1,7 g (3,7 mmoles) de l'ester éthylique précédent, on obtient après chromatographie sur colonne de silice élué avec du dichlorométhane 800 mg (50%) d' acide trans 7-[3-(1-adamantyl)-4-isopropyloxyphenyl]-3,7-diméthyl-2,4,6-heptatrienoïque de point de fusion 209-10°C.

### EXEMPLE 12

### Acide trans 7-[3-(1-adamantyl)-4-méthoxyéthoxyméthoxyphenyl]-3,7-diméthyl-2,4,6-heptatrienoïque.

(a) 3-(1-adamantyl)-4-méthoxyéthoxyméthoxyphényléthanone.
   De manière analogue à l'exemple 9(a) par réaction de 17 g (47 mmoles) d'acide 3-(1-adamantyl)-4-méthoxyéthoxyméthoxybenzoïque avec 73 ml (118 mmoles) de méthyllithuim (1,6M), on obtient 18 g (100%) du dérivé cétonique attendu sous forme d'une huile jaune.
(b) 3-méthyl-3-[3-(1-adamantyl)-4-méthoxyéthoxyméthoxyphényl]acrylonitrile.
   De manière analogue à l'exemple 9(b) par réaction de 5 g (14 mmoles) de la cétone précédente avec 3,4 ml (21 mmoles) de cyanométhylphosphonate de diéthyle, on obtient 5,8 g (100%) de nitrile attendu sous forme d'une huile jaune.
(c) 3-[3-(1-adamantyl)-4-méthoxyéthoxyméthoxyphényl]crotonaldéhyde.
   De manière analogue à l'exemple 9(c) à partir de 5,3 g (13,9 mmoles) du nitrile précédent, on obtient 2,4 g (100%) de l'aldéhyde attendu de point de fusion 80°C.
(d) trans 7-[3-(1-adamantyl)-4-méthoxyéthoxyméthoxyphenyl]-3,7-diméthyl-2,4,6-heptatrienoate d'éthyle.
   De manière analogue à l'exemple 5(d) par réaction de 1,9 g (4,9 mmoles) de 3-[3-(1-adamantyl)-4-méthoxyéthoxyméthoxyphényl]crotonaldéhyde, avec 2 ml (7,3 mmoles) de 3-méthyl-4-phosphono-2-butenoate de triéthyle, on obtient 2 g (82%) de l'ester éthylique attendu de point de fusion 83-5°C.
(e) acide trans 7-[3-(1-adamantyl)-4-méthoxyéthoxyméthoxyphenyl]-3,7-diméthyl-2,4,6-heptatrienoïque.
   De manière analogue à l'exemple 1(e) à partir de 2 g (4 mmoles) de l'ester éthylique précédent, on obtient après chromatographie sur colonne de silice élué avec un mélange d'acétate d'éthyle et de dichlorométhane (10-90) 710 mg (38%) d' acide trans 7-[3-(1-adamantyl)-4-méthoxyéthoxyméthoxyphenyl]-3,7-diméthyl-2,4,6-heptatrienoïque de point de fusion 170-2°C.

### EXEMPLE 13

### trans 7-[3-(1-adamantyl)-4-méthoxyphenyl]-7-méthyl-2,4,6-heptatrienoate d'éthyle.

(a) 3-méthyl-3-[3-(1-adamantyl)-4-méthoxyphényl]acrylonitrile.
   De manière analogue à l'exemple 9(b) par réaction de 5 g (17,6 mmoles) de 3-(1-adamantyl)-4-méthoxyphényléthanone avec 8,4 ml (52,8 mmoles) de cyanométhylphosphonate de diéthyle, on obtient 3,8 g (70%) de nitrile attendu de point de fusion 163-5°C.
(b) 3-[3-(1-adamantyl)-4-méthoxyphényl]crotonaldéhyde.
   De manière analogue à l'exemple 9(c) à partir de 7 g (22,8 mmoles) du nitrile précédent, on obtient 4,8 g (68%) de l'aldéhyde attendu de point de fusion 163-5°C.
(c) trans 7-[3-(1-adamantyl)-4-méthoxyphenyl]-7-méthyl-2,4,6-heptatrienoate d'éthyle.
   De manière analogue à l'exemple 5(d) par réaction de 500 mg (1,6 mmole) de 3-[3-(1-adamantyl)-4-méthoxyphényl]crotonaldéhyde. avec 550 µl (2,4 mmoles) de trans-4-phosphono-2-butenoate de triéthyle, on obtient 540 mg (82%) de l'ester éthylique attendu de point de fusion 143-5°C.
(d) acide trans 7-[3-(1-adamantyl)-4-méthoxyphenyl]-7-méthyl-2,4,6-heptatrienoïque.
   De manière analogue à l'exemple 1(e) à partir de 540 mg (1,3 mmoles) de l'ester éthylique précédent, on obtient après chromatographie sur colonne de silice élué avec un mélange d'acétate d'éthyle et de dichlorométhane (5-95), 220 mg (45%) d' acide trans 7-[3-(1-adamantyl)-4-méthoxyphenyl]-7-méthyl-2,4,6-heptatrienoïque.

### EXEMPLE 14

### trans 7-(3-tert-butyl-4-méthoxyéthoxyméthoxyphenyl)-3,7-diméthyl-2,4,6-heptatrienoate d'éthyle.

(a) 3-tert-butyl-4-méthoxyéthoxyméthoxy-1-bromobenzène.
   Dans un tricol et sous courant d'azote on introduit 2,4 g (81 mmoles) d'hydrure de sodium (80% dans l'huile) 50 ml de DMF, ajoute goutte à goutte une solution de 17,6 g (77 mmoles) de 2-tert-butyl-4-bromophénol dans 100 ml de DMF et agite jusqu'a cessation du dégagement gazeux. On ajoute ensuite goutte à goutte une solution de 10,5 ml (92 mmoles) de chlorure de 2-méthoxyéthoxyméthyle dans 20 ml de DMF et agite pendant quatre heures à température ambiante. On verse le milieu réactionnel dans l'eau, extrait avec de l'éther éthylique, décante la phase organique, lave à l'eau, sèche sur sulfate de magnésium, évapore. Le résidu est purifié par chromatographie sur colonne de silice élué avec un mélange de dichlorométhane et d'hexane (50-50). Après évaporation des solvants, on recueille 21,4 g (88%) du produit attendu sous forme d'une huile marron clair.
(b) acide 3-tert-butyl-4-méthoxyéthoxyméthoxybenzoïque.
   Le composé précédent 21,4 g (68 mmoles) est dissous dans 200 ml de THF et à -78°C sous courant d'azote, on ajoute goutte à goutte une solution de 30 ml de n-butyllithium (2,5 M dans l'hexane) et agite durant 30 minutes. A -78°C, on fait passer un courant de CO₂ pendant une heure et laisse remonter à température ambiante, verse le milieu réactionnel dans une solution aqueuse saturée en chlorure d'ammonium, extrait avec de l'éther éthylique, décante la phase organique, sèche sur sulfate de magnésium, évapore. Le résidu obtenu est purifié par chromatographie sur colonne de silice élué avec du dichlorométhane. On recueille 11 g (58%) de l'acide attendu.
(c) 3-tert-butyl-4-méthoxyéthoxyméthoxyphényléthanone.
   De manière analogue à l'exemple 9(a) par réaction de 11 g (39 mmoles) d'acide 3-tert-butyl-4-méthoxyéthoxyméthoxybenzoïque avec 49 ml (78 mmoles) de méthyllithuim (1,6M), on obtient 8,5 g (75%) du dérivé cétonique attendu sous forme d'une huile jaune.
(b) 3-méthyl-3-(3-tert-butyl-4-méthoxyéthoxyméthoxyphényl)acrylonitrile.
   De manière analogue à l'exemple 9(b) par réaction de 8,5 g (30 mmoles) de la cétone précédente avec 14,3 ml (90 mmoles) de cyanométhylphosphonate de diéthyle, on obtient 7 g (76%) de nitrile attendu sous forme d'une huile jaune.
(c) 3-(3-tert-butyl-4-méthoxyéthoxyméthoxyphényl)crotonaldéhyde.
   De manière analogue à l'exemple 9(c) à partir de 7 g (23 mmoles) du nitrile précèdent, on obtient 6 g (85%) de l'aldéhyde attendu sous forme d'une huile jaune.
(d) trans 7-(3-tert-butyl-4-méthoxyéthoxyméthoxyphenyl)-3,7-diméthyl-2,4,6-heptatrienoate d'éthyle.
   De manière analogue à l'exemple 5(d) par réaction de 6 g (19,6 mmoles) de 3-(3-tert-butyl-4-méthoxyéthoxyméthoxyphényl)crotonaldéhyde avec 8,1 ml (29,4 mmoles) de 3-méthyl-4-phosphono-2-butenoate de triéthyle, on obtient 6,6 g (81%) de l'ester éthylique attendu sous forme d'une huile jaune.
(e) acide trans 7-(3-tert-butyl-4-méthoxyéthoxyméthoxyphenyl)-3,7-diméthyl-2,4,6-heptatrienoïque.
   De manière analogue à l'exemple 1(e) à partir de 1,1 g (2,6 mmoles) de l'ester éthylique précédent, on obtient après recristallisation dans un mélange de dichlorométhane et d'heptane 320 mg (32%) d' acide trans 7-(3-tert-butyl-4-méthoxyéthoxyméthoxyphenyl)-3,7-diméthyl-2,4,6-heptatrienoïque de point de fusion 147-8°C.

### EXEMPLE 15

Dans cet exemple, on a illustré diverses formulations concrètes à base des composés selon l'invention.

### A- VOIE ORALE

(a) Comprimé de 0,2 g
   - Composé préparé à l'exemple 3 0,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g
(b) Suspension buvable en ampoules de 10 ml
   - Composé de l'exemple 3 0,05 g
   - Glycérine 1,000g
   - Sorbitol à 70% 1,000g
   - Saccharinate de sodium 0,010 g
   - Parahydroxybenzoate de méthyle 0,080 g
   - Arome qs
   - Eau purifiée qsp 10 ml

### B- VOIE TOPIQUE

(a) Onguent
   - Composé de l'exemple 3 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Composé de l'exemple 3 0,300 g
   - Vaseline blanche codex qsp 100 g
(c) Crème Eau-dans-Huile non ionique
   - Composé de l'exemple 3 0,100 g
   - Mélange d'alcools de lanoline émulsifs, de cires et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Composé de l'exemple 3 0,100 g
   - Polyéthylène glycol (PEG 400) 69,900 g
   - Ethanol à 95% 30,000 g
(e) Onguent hydrophobe
   - Composé de l'exemple 3 0,300 g
   - Myristate d'isopropyle 36,400 g
   - Huile de silicone ("Rhodorsil 47 V 300" vendu par RHONE-POULENC) 36,400 g
   - Cire d'abeille 13,600 g
   - Huile de silicone ("Abil 300.000 cst" vendu par GOLDSCHMIDT) qsp 100g
(f) Crème Huile-dans-Eau non ionique
   - Composé de l'exemple 3 0,500 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile qsp 100 g

## Revendications

1. Composés polyéniques, caractérisés par le fait qu'ils répondent à la formule générale (I) suivante : 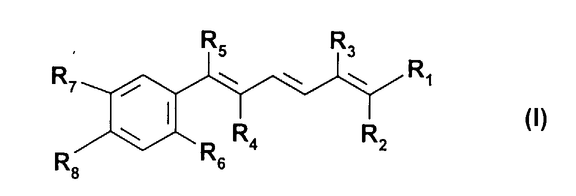 dans laquelle :
* R₁ représente un radical -CH₃, un radical -CH₂-O-R₉, un radical -CH₂-O-CO-R₁₀, ou encore un radical -CO-R₁₁, les différents radicaux R₉, R₁₀ et R₁₁ ayant la signification donnée ci-après,
* R₂ représente un atome d'hydrogène ou un radical alkyle inférieur de C₁-C₆,
* R₃ représente un atome d'hydrogène ou un radical alkyle inférieur de C₁-C₆,
* R₄ représente un atome d'hydrogène,
* R₅ représente un atome d'hydrogène ou un radical alkyle inférieur de C₁-C₆,
* R₆ représente un atome d'hydrogène, un radical alkyle, linéaire ou ramifié, ayant de 1 à 20 atomes de carbone, un radical -O-CH₂-O-CH₂-CH₂-O-CH₃ ou bien encore un radical -O-R₁₃, R₁₃ ayant la signification donnée ci-après,
* R₇ et R₈ représentent indépendamment (i) un atome d'hydrogène, (ii) un radical alkyle, linéaire ou ramifié, ayant de 1 à 20 atomes de carbone, (iii) un radical cycloaliphatique, (iv) un radical -O-CH₂-O-CH₂-CH₂-O-CH₃, (v) un radical -O- R₁₃, ou bien encore (vi) un radical -S(O)ₙR₁₃, R₁₃ et n ayant la signification donnée ci-après,
étant entendu que dans tout ce qui précède :
- R₄ et R₆, pris ensemble, peuvent former avec le cycle benzénique adjacent un cycle naphtalénique,
- l'un au moins des radicaux R₇ ou R₈ représente un radical (ii) ou (iii) , avec la condition que lorsque l'un représente le radical (ii), l'autre ne représente pas un radical (ii),
- R₉ représente un atome d'hydrogène ou un radical alkyle inférieur de C₁-C₆,
- R₁₀ représente un radical alkyle inférieur de C₁-C₆,
- R₁₁ représente.
(a) un atome d'hydrogène
(b) un radical alkyle inférieur de C₁-C₆
(c) un radical de formule : 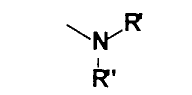 dans laquelle R' et R" représentent indépendamment un atome d'hydrogène, un radical alkyle inférieur de C₁-C₆, un radical mono ou polyhydroxyalkyle, un radical aryle éventuellement substitué o.u un reste d'aminoacide ou de peptide ou de sucre ou encore, pris ensemble, forment un hétérocycle,
(d) un radical -OR₁₂, R₁₂ représentant un atome d'hydrogène, un radical alkyle linéaire ou ramifié ayant de 1 à 20 atomes de carbone, un radical alkényle, un radical mono ou polyhydroxyalkyle, un radical aryle ou aralkyle éventuellement substitué ou un reste de sucre ou un reste d'aminoacide ou de peptide,
- R₁₃ représente un atome d'hydrogène ou un radical alkyle, linéaire ou ramifié, ayant de 1 à 20 atomes de carbone,
- n est un nombre entier égal à 0, 1 ou 2,
ainsi que leurs sels, et leurs isomères optiques et géométriques.

2. Composés selon la revendication 1, caractérisés en ce qu'ils se présentent sous forme de sels d'un métal alcalin ou alcalino-terreux, ou encore de zinc ou d'une amine organique.

3. Composés selon l'une des revendications 1 ou 2, caractérisés en ce que les radicaux alkyles inférieurs sont choisis dans le groupe constitué par les radicaux méthyle, éthyle, isopropyle, butyle, tertiobutyle et hexyle.

4. Composés selon l'une quelconque des revendications précédentes, caractérisés en ce que les radicaux alkyles linéaires ou ramifiés ayant de 1 à 20 atomes de carbone sont choisis dans le groupe constitué par les radicaux méthyle, éthyle, propyle, 2-éthyl-hexyle, octyle, dodécyle, hexadécyle et octadécyle.

5. Composés selon l'une quelconque des revendications précédentes, caractérisés en ce que les radicaux monohydroxyalkyles sont choisis dans le groupe constitué par les radicaux 2-hydroxyéthyle, 2-hydroxypropyle ou 3-hydroxypropyle.

6. Composés selon l'une quelconque des revendications précédentes, caractérisés en ce que les radicaux polyhydroxyalkyles sont choisis dans le groupe constitué par les radicaux 2,3-dihydroxypropyle, 2,3,4-trihydroxybutyle, 2,3,4,5-tétrahydroxypentyle ou le reste du pentaérythritol.

7. Composés selon l'une quelconque des revendications précédentes, caractérisés en ce que le radical aryle est un radical phényle éventuellement substitué par au moins un atome d'halogène, un hydroxyle ou une fonction nitro.

8. Composés selon l'une quelconque des revendications précédentes, caractérisés en ce que les radicaux aralkyles sont choisis dans le groupe constitué par les radicaux benzyle ou phénéthyle éventuellement substitués par au moins un atome d'halogène, un hydroxyle ou une fonction nitro.

9. Composés selon l'une quelconque des revendications précédentes, caractérisés en ce que les radicaux alkényles sont choisis dans le groupe constitué par les radicaux contenant de 2 à 5 atomes de carbone et présentant une ou plusieurs insaturations éthyléniques, en particulier le radical allyle.

10. Composés selon l'une quelconque des revendications précédentes, caractérisés en ce que les restes de sucre sont choisis dans le groupe constitué par les restes de glucose, de galactose, de mannose ou d'acide glucuronique.

11. Composés selon l'une quelconque des revendications précédentes, caractérisés en ce que les restes d'aminoacide sont choisis dans le groupe constitué par les restes dérivant de la lysine, de la glycine ou de l'acide aspartique.

12. Composés selon l'une quelconque des revendications précédentes, caractérisés en ce que les restes de peptides sont choisis dans le groupe constitué par les restes de dipeptides ou de tripeptides.

13. Composés selon l'une quelconque des revendications précédentes, caractérisés en ce que les radicaux hétérocycliques sont choisis dans le groupe constitué par les radicaux pipéridino, morpholino, pyrrolidino ou pipérazino, éventuellement substitués en position 4 par un radical alkyle en C₁-C₆ ou mono ou polyhydroxyalkyle.

14. Composés selon l'une quelconque des revendications précédentes, caractérisés en ce que les radicaux cycloaliphatiques sont choisis dans le groupe constitué par les radicaux 1-méthyl cyclohexyle et 1-adamantyle.

15. Composés selon la revendication 1, caractérisés par le fait qu'ils sont pris, seuls ou en mélanges, dans le groupe constitué par :
- Acide *trans* 7-[3-(1-adamantyl)-4-hydroxyphenyl]-3,7-diméthyl-2,4,6-heptatrienoïque
Ethyl *trans* 7-[3-(1-adamantyl)-4-méthoxyphenyl]-3,7-diméthyl-2,4,6-heptatrienoate
Acide trans 7-[3-(1-adamantyl)-4-méthoxyphenyl]-3,7-diméthyl-2,4,6-heptatrienoïque
Acide (2Z,4E,6E) 7-[3-(1-adamantyl)-4-méthoxyphenyl]-3,7-diméthyl-2,4,6-heptatrienoïque.
Acide *trans* 5-[-7-(1-adamantyl)-6-méthoxyéthoxyméthoxy-2-naphtyl]-3-méthyll-2,4-pentadienoïque.
Acide *trans* 5-[-7-(1-adamantyl)-6-hydroxy-2-naphtyl]-3-méthyll-2,4-pentadienoïque.
Acide *trans* 5-[-7-(1-adamantyl)-6-méthoxy-2-naphtyl]-3-méthyll-2,4-pentadienoïque.
Acide *trans* 5-[-7-(1-adamantyl)-6-propyloxy-2-naphtyl]-3-méthyll-2,4-pentadienoïque.
Acide *trans* 7-[3-(1-méthylcyclohexyl)-4-hydroxyphenyl]-3,7-diméthyl-2,4,6-heptatrienoïque.
Acide (2Z,4E,6E) 7-[3-(1-méthylcyclohexyl)-4-méthoxyphenyl]-3,7-diméthyl-2,4,6-heptatrienoïque.
Acide *trans* 7-[3-(1-adamantyl)-4-isopropyloxyphenyl]-3,7-diméthyl-2,4,6-heptatrienoïque.
acide *trans* 7-[3-(1-adamantyl)-4-méthoxyéthoxyméthoxyphenyl]-3,7-diméthyl-2,4,6-heptatrienoïque.
acide *trans* 7-[3-(1-adamantyl)-4-méthoxyphenyl]-7-méthyl-2,4,6-heptatrienoïque.
*trans* 7-[3-(1-adamantyl)-4-méthoxyphenyl]-3,7-diméthyl-2,4,6-heptatrien-1-ol.
*trans* 7-[3-(1-adamantyl)-4-méthoxyphenyl]-3,7-diméthyl-2,4,6-heptatrien-1-al.
*trans* N-éthyl-7-[3-(1-adamantyl)-4-méthoxyphenyl]-3,7-diméthyl-2,4,6-heptatrienecarboxamide.
*trans* N-4-hydroxyphényl-7-[3-(1-adamantyl)-4-méthoxyphenyl]-3,7-diméthyl-2,4,6-heptatrienecarboxamide.
acide *trans* 7-(3-tert-butyl-4-méthoxyéthoxyméthoxyphenyl)-3,7-diméthyl-2,4,6-heptatrienoïque.
*trans* 7-(3-tert-butyl-4-hydroxyphenyl)-3,7-diméthyl-2,4,6-heptatrienoate d'éthyle.
*trans* 7-(3-tert-butyl-4-méthoxyphenyl)-3,7-diméthyl-2,4,6-heptatrienoate d'éthyle.
*trans* 7-[4-(1-adamantyl)-3-méthoxyphenyl]-3,7-diméthyl-2,4,6-heptatrienoate d'éthyle.

16. Composés selon la revendication 1, caractérisés par le fait qu'ils présentent l'une au moins, et de préférence toutes, les caractéristiques suivantes : R₁ est un radical -CO-R₁₁ ; R₇ est le radical adamantyl ; R₄ est un atome d'hydrogène ; R₆ est un atome d'hydrogène ou un radical alkyl, linéaire ou ramifié, ayant de 1 à 20 atomes de carbone ; R₄ et R₆ forment avec le cycle benzénique adjacent un cycle naphthalénique.

17. Composés selon l'une quelconque des revendications précédentes pour une utilisation comme médicament.

18. Composés selon la revendication 17 pour une utilisation comme médicament destiné au traitement des affections dermatologiques, rhumatismales, respiratoires, cardiovasculaires et ophtalmologiques, ainsi qu'au traitement ou à la prévention de l'ostéoporose.

19. Utilisation de l'un au moins des composés définis aux revendications 1 à 16 pour la fabrication d'un médicament destiné au traitement des affections dermatologiques, rhumatismales, respiratoires, cardiovasculaires et ophtalmologiques, ainsi qu'au traitement ou à la prévention de l'ostéoporose.

20. Composition pharmaceutique, caractérisée par le fait qu'elle comprend, dans un support pharmaceutiquement acceptable, au moins l'un des composés tels que définis à l'une quelconque des revendications 1 à 16.

21. Composition selon la revendication 20, caractérisée en ce que la concentration en composé(s) selon l'une des revendication 1 à 16 est comprise entre 0,001 % et 5 % en poids par rapport à l'ensemble de la composition.

22. Composition cosmétique, caractérisée par le fait qu'elle comprend, dans un support cosmétiquement acceptable, au moins l'un des composés tels que définis à l'une quelconque des revendications 1 à 16.

23. Composition selon la revendication 22, caractérisée en ce que la concentration en composé(s) selon l'une des revendications 1 à 16 est comprise entre 0,001 % et 3 % en poids par rapport à l'ensemble de la composition.

24. Utilisation d'une composition cosmétique telle que définie à l'une des revendications 22 ou 23 pour l'hygiène corporelle ou capillaire.

## Patentansprüche

1. Polyenverbindungen,
**dadurch gekennzeichnet, daß**
sie der folgenden allgemeinen Formel (I) entsprechen: 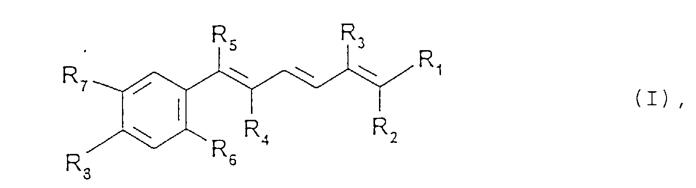 worin bedeuten:
* R₁ -CH₃, -CH₂-O-R₉, -CH₂-O-CO-R₁₀ oder -CO-Rₗ₁, wobei die verschiedenen Gruppen R₉, R₁₀ und R₁₁ die nachfolgend angegebenen Bedeutungen aufweisen,
* R₂ Wasserstoff oder eine niedere C₁₋₆-Alkylgruppe,
* R₃ Wasserstoff oder eine niedere C₁₋₆-Alkylgruppe,
* R₄ Wasserstoff,
* R₅ Wasserstoff oder eine niedere C₁₋₆-Alkylgruppe,
* R₆ Wasserstoff, eine geradkettige oder verzweigte Alkylgruppe mit 1 bis 20 Kohlenstoffatomen, -O-CH₂-O-CH₂-CH₂-O-CH₃ oder auch -O-R₁₃, wobei R₁₃ die nachfolgend angegebene Bedeutung aufweist,
* R₇ und R₈ unabhängig voneinander (i) Wasserstoff, (ii) eine geradkettige oder verzweigte Alkylgruppe mit 1 bis 20 Kohlenstoffatomen, (iii) eine cycloaliphatische Gruppe, (iv) -O-CH₂-O-CH₂-CH₂-O-CH₃, (v) eine Gruppe -O-R₁₃ oder auch (vi) eine Gruppe -S(O)ₙR₁₃, wobei R₁₃ und n die nachfolgend angegebenen Bedeutungen aufweisen,
mit der Maßgabe, daß im folgenden:
- R₄ und R₆ gemeinsam mit dem angrenzenden Benzolring einen Naphthalinring bilden können,
- mindestens eine der beiden Gruppen R₇ oder R₈ eine Gruppe (ii) oder (iii) bedeutet, mit der Maßgabe, daß, wenn eine der Gruppen die Gruppe (ii) bedeutet, die andere Gruppe nicht die Gruppe (ii) bedeuten kann,
- R₉ Wasserstoff oder eine niedere C₁₋₆-Alkylgruppe bedeutet,
- R₁₀ eine niedere C₁₋₆-Alkylgruppe bedeutet,
- R₁₁ bedeutet:
(a) ein Wasserstoffatom,
(b) eine niedere C₁₋₆-Alkylgruppe,
(c) eine Gruppe der Formel: 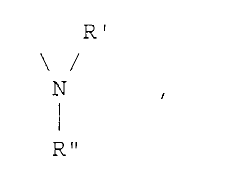 worin R' und R" unabhängig voneinander ein Wasserstoffatom, eine niedere C₁₋₆-Alkylgruppe, eine Mono- oder Polyhydroxyalkylgruppe, eine gegebenenfalls substituierte Arylgruppe oder einen Aminosäure-, Peptid- oder Zuckerrest bedeuten, oder gemeinsam einen Heterocyclus bilden,
d) eine Gruppe -OR₁₂, wobei R₁₂ Wasserstoff, stoff, eine geradkettige oder verzweigte Alkylgruppe mit 1 bis 20 Kohlenstoffatomen, eine Alkinylgruppe, eine Mono- oder Polyhydroxyalkylgruppe, eine gegebenenfalls substituierte Aryl- oder Aralkylgruppe oder einen Zucker-, Aminosäure- oder Peptidrest bedeutet,
- R₁₃ ein Wasserstoffatom oder eine geradkettige oder verzweigte Alkylgruppe mit 1 bis 20 Kohlenstoffatomen bedeutet, und
- n Null, 1 oder 2 ist,
sowie deren Salze und deren optischen und geometrischen Isomere.

2. Verbindungen nach Anspruch 1, dadurch gekennzeichnet, daß sie in Form von Alkali- oder Erdalkalimetallsalzen, Zinksalzen oder auch Salzen eines organischen Amins vorliegen.

3. Verbindungen nach einem der Ansprüche 1 oder 2, dadurch gekennzeichnet, daß die niederen Alkylgruppen unter Methyl, Ethyl, Isopropyl, Butyl, tert.-Butyl und Hexyl ausgewählt sind.

4. Verbindungen nach einem der vorhergehenden Ansprüche, dadurch gekennzeichnet, daß die geradkettigen oder verzweigten Alkylgruppen mit 1 bis 20 Kohlenstoffatomen unter Methyl, Ethyl, Propyl, 2-Ethylhexyl, Octyl, Dodecyl, Hexadecyl und Octadecyl ausgewählt sind.

5. Verbindungen nach einem der vorhergehenden Ansprüche, dadurch gekennzeichnet, daß die Monohydroxyalkylgruppen unter 2-Hydroxyethyl, 2-Hydroxypropyl oder 3-Hydroxypropyl ausgewählt sind.

6. Verbindungen nach einem der vorhergehenden Ansprüche, dadurch gekennzeichnet, daß die Polyhydroxyalkylgruppen unter 2,3-Dihydroxypropyl, 2,3,4-Trihydroxybutyl, 2,3,4,5-Tetrahydroxypentyl oder der Pentaerythritgruppe ausgewählt sind.

7. Verbindungen nach einem der vorhergehenden Ansprüche, dadurch gekennzeichnet, daß die Arylgruppe unter einer Phenylgruppe ausgewählt ist, die gegebenenfalls mit mindestens einem Halogenatom, einer Hydroxygruppe oder einer Nitrogruppe substituiert ist.

8. Verbindungen nach einem der vorhergehenden Ansprüche, dadurch gekennzeichnet, daß die Aralkylgruppen unter Benzyl oder Phenethyl ausgewählt sind, die gegebenenfalls mit mindestens einem Halogenatom, einer Hydroxygruppe oder einer Nitrogruppe substituiert sind.

9. Verbindungen nach einem der vorhergehenden Ansprüche, dadurch gekennzeichnet, daß die Alkenylgruppen unter den Gruppen mit 2 bis 5 Kohlenstoffatomen ausgewählt sind, die eine oder mehrere ethylenisch ungesättigte Doppelbindungen aufweisen, insbesondere der Allylgruppe.

10. Verbindungen nach einem der vorhergehenden Ansprüche, dadurch gekennzeichnet, daß die Zuckerreste unter den Glucose-, Galactose-, Mannose- oder Glucuronsäureresten ausgewählt sind.

11. Verbindungen nach einem der vorhergehenden Ansprüche, dadurch gekennzeichnet, daß die Aminosäurereste unter den Resten ausgewählt sind, die von Lysin, Glycin oder Asparaginsäure abgeleitet sind.

12. Verbindungen nach einem der vorhergehenden Ansprüche, dadurch gekennzeichnet, daß die Peptidreste unter den Dipeptid- oder Tripeptidresten ausgewählt sind.

13. Verbindungen nach einem der vorhergehenden Ansprüche, dadurch gekennzeichnet, daß die heterocyclischen Gruppen unter Piperidino, Morpholino, Pyrrolidino oder Piperazino ausgewählt sind, die gegebenenfalls in 4-Stellung mit einer C₁₋₆-Alkylgruppe oder Mono- oder Polyhydroxyalkylgruppe substituiert sind.

14. Verbindungen nach einem der vorhergehenden Ansprüche, dadurch gekennzeichnet, daß die cycloaliphatischen Gruppen unter 1-Methylcyclohexyl und 1-Adamantyl ausgewählt sind.

15. Verbindungen nach Anspruch 1, dadurch gekennzeichnet, daß sie aus den folgenden Verbindungen oder deren Gemischen ausgewählt sind:
*trans*-7-[3-(1-Adamantyl)-4-hydroxyphenyl]-3,7-dimethyl-2,4,6-heptatriensäure
*trans*-7-[3-(1-Adamantyl)-4-methoxyphenyl]-3,7-dimethyl-2,4,6-heptatriensäureethylester,
*trans*-7-[3-(1-Adamantyl)-4-methoxyphenyl]-3,7-dimethyl-2,4,6-heptatriensäure
- (2Z,4E,6E)-7-(1-Adamantyl)-4-methoxyphenyl]-3,7-dimethyl-2,4,6-heptatriensäure
trans-5- [7-(1-Adamantyl)-6-methoxyethoxymethoxy-2-naphthyl]-3-methyl-2,4-pentadiensäure
trans-5- [7-(1-Adamantyl)-6-hydroxy-2-naphthyl]-3-methyl-2,4-pentadiensäure
*trans*-5-[7-(1-Adamantyl)-6-methoxy-2-naphthyl]-3-methyl-2,4-pentadiensäure
*trans*-5-[7-(1-Adamantyl)-6-propyloxy-2-naphthyl]-3-methyl-2,4-pentadiensäure
*trans*-7-[3-(1-methylcyclohexyl)-4-hydroxyphenyl]-3,7-dimethyl-2,4,6-heptadiensäure
(2Z,4E,6E)-7-[3-(1-methylcyclohexyl)-4-methoxyphenyl]-3,7-dimethyl-2,4,6-heptatriensäure
trans-7- 3-(1-Adamantyl)-4-isopropyloxyphenyl]-3,7-dimethyl-2,4,6-heptatriensäure
*trans*-7-[3-(1-Adamantyl)-4-methoxyethoxymethoxyphenyl]-3,7-dimethyl-2,4,6-heptatriensäure
*trans*-7-[3-(1-Adamantyl)-4-methoxyphenyl]-7-methyl-2,4,6-heptatriensäure
trans-7-[3-(1-Adamantyl)-4-methoxyphenyl]-3,7-dimethyl-2,4,6-heptatrien-1-ol
*trans*-7-[3-(1-Adamantyl]-4-methoxyphenyl]-3,7-dimethyl-2,4,6-heptatrien-1-al
*trans*-N-Ethyl-7-[3-adamantyl)-4-methoxyphenyl]-3,7-dimethyl-2,4,6-heptatriencarboxamid
*trans*-N-4-Hydroxyphenyl-7-[3-(1-adamantyl)-4-methoxyphenyl]-3,7-dimethyl-2,4,6-heptatriencarboxamid
*trans*-7-(3-tert.-Butyl-4-methoxyethoxymethoxyphenyl)-3,7-dimethyl-2,4,6-heptatriensäure
*trans*-7-(3-tert.-Butyl-4-hydroxyphenyl)-3,7-dimethyl-2,4,6-heptatriensäureethylester
*trans*-7-(3-*tert*.-Butyl-4-methoxyphenyl)-3,7-dimethyl-2,4,6-heptatriensäureethylester
*trans*-7-[4-(1-Adamantyl)-3-methoxyphenyl]-3,7-dimethyl-2,4,6-heptatriensäureethylester.

16. Verbindungen nach Anspruch 1, dadurch gekennzeichnet, daß sie mindestens eine und vorzugsweise alle folgenden Eigenschaften aufweisen: R₁ ist -CO-R₁₁; R₇ ist Adamantyl; R₄ bedeutet Wasserstoff; R₆ ist ein Wasserstoffatom oder eine geradkettige oder verzweigte Alkylgruppe mit 1 bis 20 Kohlenstoffatomen; R₄ und R₆ bilden gemeinsam mit dem angrenzenden Benzolring einen Naphthalinring.

17. Verbindungen nach einem der vorhergehenden Ansprüche zur Verwendung als Arzneimittel.

18. Verbindungen nach Anspruch 17 für eine Verwendung als Arzneimittel, das zur Behandlung von dermatologischen Erkrankungen, rheumatischen Erkrankungen, Erkrankungen der Atemwege, kardiovaskulären Erkrankungen und ophthalmologischen Erkrankungen bestimmt ist, sowie zur Behandlung oder zur Vorbeugung von Osteoporose.

19. Verwendung mindestens einer Verbindung nach einem der Ansprüche 1 bis 16 zur Herstellung eines Arzneimittels, das zur Behandlung von dermatologischen Erkrankungen, rheumatischen Erkrankungen, Erkrankungen der Atemwege, kardiovaskulären Erkrankungen und ophthalmologischen Erkrankungen sowie zur Behandlung oder zur Vorbeugung von Osteoporose bestimmt ist.

20. Pharmazeutische Zusammensetzung, dadurch gekennzeichnet, daß sie in einem pharmazeutisch akzeptablen Träger mindestens eine der Verbindungen nach einem der Ansprüche 1 bis 16 enthält.

21. Zusammensetzung nach Anspruch 20, dadurch gekennzeichnet, daß die Konzentration der Verbindung(en) nach einem der Ansprüche 1 bis 16 im Bereich von 0,001 bis 5 Gew.-%, bezogen auf die gesamte Zusammensetzung, liegt.

22. Kosmetische Zusammensetzung, dadurch gekennzeichnet, daß sie in einem kosmetisch akzeptablen Träger mindestens eine Verbindung nach einem der Ansprüche 1 bis 16 enthält.

23. Zusammensetzung nach Anspruch 22, dadurch gekennzeichnet, daß die Verbindung(en) nach einem der Ansprüche 1 bis 16 im Bereich von 0,001 bis 3 Gew.-%, bezogen auf die gesamte Zusammensetzung, liegt.

24. Verwendung einer kosmetischen Zusammensetzung nach einem der Ansprüche 22 oder 23 zur Körper- oder Haarhygiene.

## Claims

1. Polyenic compounds, characterized in that they correspond to the general formula (I) below: 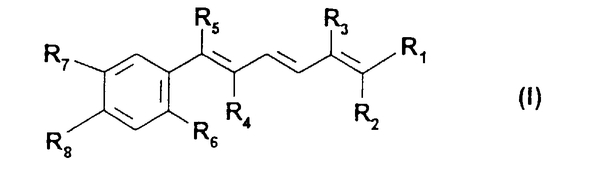 in which
* R₁ represents a -CH₃ radical, a radical -CH₂-O-R₉, a radical -CH₂-O-CO-R₁₀, or alternatively a radical -CO-R₁₁, the various radicals R₉, R₁₀ and R₁₁ having the meaning given below,
* R₂ represents a hydrogen atom or a lower C₁-C₆ alkyl radical,
* R₃ represents a hydrogen atom or a lower C₁-C₆ alkyl radical,
* R₄ represents a hydrogen atom,
* R₅ represents a hydrogen atom or a lower C₁-C₆ alkyl radical,
* R₆ represents a hydrogen atom, a linear or branched alkyl radical having from 1 to 20 carbon atoms, a radical -O-CH₂-O-CH₂-CH₂-O-CH₃ or alternatively a radical -O-R₁₃, R₁₃ having the meaning given below,
* R₇ and R₈ independently represent (i) a hydrogen atom, (ii) a linear or branched alkyl radical having from 1 to 20 carbon atoms, (iii) a cycloaliphatic radical, (iv) a radical -O-CH₂-O-CH₂-CH₂-O-CH₃, (v) a radical -O-R₁₃, or alternatively (vi) a radical -S(O)ₙR₁₃, R₁₃ and n having the meaning given below,
it being understood that, in all of the preceding text:
- R₄ and R₆, taken together, may form, with the adjacent benzene ring, a naphthalene ring,
- at least one of the radicals R₇ or R₈ represents a radical (ii) or (iii), with the condition that, when one represents the radical (ii), the other does not represent a radical (ii),
- R₉ represents a hydrogen atom or a lower C₁-C₆ alkyl radical
- R₁₀ represents a lower C₁-C₆ alkyl radical
- R₁₁ represents:
(a) a hydrogen atom
(b) a lower C₁-C₆ alkyl radical
(c) a radical of formula: 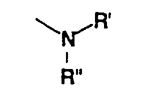 in which R' and R" independently represent a hydrogen atom, a lower C₁-C₆ alkyl radical, a mono- or polyhydroxyalkyl radical, an optionally substituted aryl radical or an amino acid residue or peptide residue or sugar residue or alternatively, taken together, they form a heterocycle,
(d) a radical -OR₁₂, R₁₂ representing a hydrogen atom, a linear or branched alkyl radical having from 1 to 20 carbon atoms, an alkenyl radical, a mono- or polyhydroxyalkyl radical, an optionally substituted aryl or aralkyl radical or a sugar residue or an amino acid residue or peptide residue,
- R₁₃ represents a hydrogen atom or a linear or branched alkyl radical having from 1 to 20 carbon atoms,
- and n is an integer equal to 0, 1 or 2,
as well as the salts and the optical and geometrical isomers thereof.

2. Compounds according to Claim 1, characterized in that they are in the form of alkali metal salts or an alkaline-earth metal salts, or alternatively in the form of zinc salts or organic amine salts.

3. Compounds according to either of Claims 1 and 2, characterized in that the lower alkyl radicals are chosen from the group consisting of the methyl, ethyl, isopropyl, butyl, tert-butyl and hexyl radicals.

4. Compounds according to any one of the preceding claims, characterized in that the linear or branched alkyl radicals having from 1 to 20 carbon atoms are chosen from the group consisting of the methyl, ethyl, propyl, 2-ethylhexyl, octyl, dodecyl, hexadecyl and octadecyl radicals.

5. Compounds according to any one of the preceding claims, characterized in that the monohydroxyalkyl radicals are chosen from the group consisting of the 2-hydroxyethyl, 2-hydroxypropyl and 3-hydroxypropyl radicals.

6. Compounds according to any one of the preceding claims, characterized in that the polyhydroxyalkyl radicals are chosen from the group consisting of the 2,3-dihydroxypropyl, 2,3,4-trihydroxybutyl and 2,3,4,5-tetrahydroxypentyl radicals or the pentaerythritol residue.

7. Compounds according to any one of the preceding claims, characterized in that the aryl radical is a phenyl radical which is optionally substituted with at least one halogen atom, one hydroxyl or one nitro function.

8. Compounds according to any one of the preceding claims, characterized in that the aralkyl radicals are chosen from the group consisting of benzyl or phenethyl radicals which are optionally substituted with at least one halogen atom, one hydroxyl or one nitro function.

9. Compounds according to any one of the preceding claims, characterized in that the alkenyl radicals are chosen from the group consisting of the radicals containing from 2 to 5 carbon atoms and having one or more ethylenic unsaturations, in particular the allyl radical.

10. Compounds according to any one of the preceding claims, characterized in that the sugar residues are chosen from the group consisting of the glucose, galactose, mannose or glucuronic acid residues.

11. Compounds according to any one of the preceding claims, characterized in that the amino acid residues are chosen from the group consisting of the residues derived from lysine, from glycine or from aspartic acid.

12. Compounds according to any one of the preceding claims, characterized in that the peptide residues are chosen from the group consisting of dipeptide and tripeptide residues.

13. Compounds according to any one of the preceding claims, characterized in that the heterocyclic radicals are chosen from the group consisting of the piperidino, morpholino, pyrrolidino and piperazino radicals, which are optionally substituted at position -4 with a C₁-C₆ alkyl radical or a mono- or polyhydroxyalkyl radical.

14. Compounds according to any one of the preceding claims, characterized in that the cycloaliphatic radicals are chosen from the group consisting of the 1-methylcyclohexyl and 1-adamantyl radicals.

15. Compounds according to Claim 1, characterized in that they are taken, alone or as mixtures, from the group consisting of:
*trans*-7-[3-(1-Adamantyl)-4-hydroxyphenyl]-3,7-dimethyl-2,4,6-heptatrienoic acid.
Ethyl *trans*-7-[3-(1-adamantyl)-4-methoxyphenyl]-3,7-dimethyl-2,4,6-heptatrienoate.
*trans*-7- [3- (1-Adamantyl)-4-methoxyphenyl]-3,7-dimethyl-2,4,6-heptatrienoic acid.
(2Z,4E,6E)-7-[3-(1-Adamantyl)-4-methoxyphenyl]-3,7-dimethyl-2,4,6-heptatrienoic acid.
*trans*-5-[-7-(1-Adamantyl)-6-methoxyethoxymethoxy2-naphthyl]-3-methyl-2,4-pentadienoic acid.
*trans*-5-[-7-(1-Adamantyl)-6-hydroxy-2-naphthyl]-3-methyl-2,4-pentadienoic acid.
*trans*-5-[-7-(1-Adamantyl)-6-methoxy-2-naphthyl] 3-methyl-2,4-pentadienoic acid.
*trans*-5-[-7-(1-Adamantyl)-6-propyloxy-2-naphthyl]-3-methyl-2,4-pentadienoic acid.
*trans*-7-[3-(1-Methylcyclohexyl)-4-hydroxyphenyl]-3,7-dimethyl-2,4,6-heptatrienoic acid.
(2Z,4E,6E)-7-[3-(1-Methylcyclohexyl)-4-methoxyphenyl]-3,7-dimethyl-2,4,6-heptatrienoic acid.
*trans*-7-[3-(1-Adamantyl)-4-isopropyloxy-phenyl]- 3,7-dimethyl-2,4,6-heptatrienoic acid.
*trans*-7-[3-(1-Adamantyl)-4-methoxyethoxy-methoxyphenyl]-3,7-dimethyl-2,4,6-heptatrienoic acid.
*trans*-7-[3-(1-Adamantyl)-4-methoxyphenyl]-7-methyl-2,4,6-heptatrienoic acid.
*trans*-7-[3-(1-Adamantyl)-4-methoxyphenyl]-3,7-dimethyl-2,4,6-heptatrien-1-ol.
*trans*-7-[3-(1-Adamantyl)-4-methoxyphenyl]-3,7-dimethyl-2,4,6-heptatrien-1-al.
*trans*-N-Ethyl-7-[3-(1-adamantyl)-4-methoxyphenyl]-3,7-dimethyl-2,4,6-heptatrienecarboxamide.
*trans*-N-4-Hydroxyphenyl-7-[3-(1-adamantyl)-4-methoxyphenyl]-3,7-dimethyl-2,4,6-heptatrienecarboxamide.
*trans*-7-(3-tert-Butyl-4-methoxyethoxymethoxyphenyl)-3,7-dimethyl-2,4,6-heptatrienoic acid.
Ethyl *trans*-7-(3-tert-butyl-4-hydroxyphenyl)-3,7-dimethyl-2,4,6-heptatrienoate.
Ethyl *trans*-7-(3-tert-butyl-4-methoxyphenyl)-3,7-dimethyl-2,4,6-heptatrienoate.
Ethyl *trans*-7-[4-(1-adamantyl)-3-methoxyphenyl]-3,7-dimethyl-2,4,6-heptatrienoate.

16. Compounds according to Claim 1, characterized in that they have at least one, and preferably all, of the following characteristics: R₁ is a radical -CO-R₁₁; R₇ is the adamantyl radical; R₄ is a hydrogen atom; R₆ is a hydrogen atom or a linear or branched alkyl radical having from 1 to 20 carbon atoms; R₄ and R₆ form, with the adjacent benzene ring, a naphthalene ring.

17. Compounds according to any one of the preceding claims, for use as a medicament.

18. Compounds according to Claim 17, for use as a medicament intended for the treatment of dermatological, rheumatic, respiratory, cardiovascular and ophthalmological complaints, as well as for the treatment or the prevention of osteoporosis.

19. Use of at least one of the compounds defined in Claims 1 to 16 for the manufacture of a medicament intended for the treatment of dermatological, rheumatic, respiratory, cardiovascular and ophthalmological complaints, as well as for the treatment or the prevention of osteoporosis.

20. Pharmaceutical composition, characterized in that it comprises, in a pharmaceutically acceptable vehicle, at least one of the compounds as defined in any one of Claims 1 to 16.

21. Composition according to Claim 20, characterized in that the concentration of compound(s) according to one of Claims 1 to 16 is between 0.001 % and 5 % by weight relative to the whole composition.

22. Cosmetic composition, characterized in that it comprises, in a cosmetically acceptable vehicle, at least one of the compounds as defined in any one of Claims 1 to 16.

23. Composition according to Claim 22, characterized in that the concentration of compound(s) according to one of Claims 1 to 16 is between 0.001 % and 3 % by weight relative to the whole composition.

24. Use of a cosmetic composition as defined in either of Claims 22 and 23, for body or hair hygiene.
